# EUROPEAN PATENT APPLICATION

(11) **EP 4 691 523 A1**
(43) Date of publication of application: **11.02.2026**
(21) Application number: 24780514.6
(22) Date of filing: 27.03.2024
(51) Int. Cl.: A61M 5/315, A61L 29/08, A61L 31/10, A61M 5/28

(54) **MEDICAL INSTRUMENT, TREATMENT SOLUTION, AND METHOD FOR MANUFACTURING MEDICAL INSTRUMENT**

(30) Priority: 31.03.2023 JP 2023058436
(71) Applicant: JMS Co., Ltd., Hiroshima 730-8652 (JP)
(72) Inventor: INOUE Yuya, Hiroshima-shi, Hiroshima 730-8652 (JP); YAMASHINA Yuka, Hiroshima-shi, Hiroshima 730-8652 (JP)
(74) Representative: Berggren Oy
(86) International application number: PCT/JP2024/012387
(87) International publication number: WO 2024/204412

(57) **Abstract**

Provided are: a medical instrument having excellent slidability and having a sliding part in which peeling of a coating film provided in the sliding part is suppressed; a chemical solution suitably used for manufacturing the medical instrument; and a method for manufacturing the medical instrument, the method comprising forming the coating film using the chemical solution. In a medical instrument provided with a sliding part, a coating film comprising a cured product of a composition containing a silicone resin having a hydroxyl group and a silane coupling agent is formed on the surface of the sliding part. The composition contains an aminosilane having a specific structure as a silane coupling agent or an amine compound not corresponding to the aminosilane, and also contains a haloalkylsilane having a specific structure.

## Description

### TECHNICAL FIELD

The present invention relates to a medical device, a treatment liquid, and a method for producing a medical device.

### BACKGROUND ART

Many medical devices are required to have high glide properties, such as syringe barrels, plungers, injection needles, and bottle needles. A known method for imparting high glide properties to a medical device includes applying a lubricant to a target portion of the medical device in need of such properties. Specifically, a proposed method includes applying a small amount of a silicone compound to a laminated film covering a stopper (gasket) at the top of the plunger of a prefilled syringe (see Patent Document 1).

### Citation List

### Patent Document

Patent Document 1: Japanese Unexamined Patent Application, Publication No. 2020-182677

### DISCLOSURE OF THE INVENTION

### Problems to be Solved by the Invention

When a certain chemical is used to form a coating on a medical device, the coating is required to be resistant to delamination from the medical device so that it will have no adverse effect on the drug solution or the human body. In the prefilled syringe of Patent Document 1, the coating of a small amount of the silicone compound is less likely to delaminate from the stopper surface. Unfortunately, due to such a small amount of the silicone compound coating, the prefilled syringe of Patent Document 1 has room for improvement in glide properties.

It is an object of the present invention, which has been made in view of the problem described above, to provide a medical device including a sliding portion having a coating that has high glide properties and is resistant to delamination; a chemical solution suitable for use in production of such a medical device; and a medical device production method that includes forming a coating using such a chemical solution.

### Means for Solving the Problems

The inventors have completed the present invention based on findings that a solution to the problem can be provided when a coating is formed on the surface of the sliding portion of a medical device by applying and curing a composition including: a hydroxy group-containing silicone resin; at least one silane coupling agent including an aminosilane with a specific structure; and a haloalkylsilane with a specific structure; and optionally a non-aminosilane amine compound.

### Effects of the Invention

The present invention provides a medical device including a sliding portion having a coating that has high glide properties and is resistant to delamination; a chemical solution suitable for use in production of such a medical device; and a medical device production method that includes forming a coating using such a chemical solution.

### PREFERRED MODE FOR CARRYING OUT THE INVENTION

### <<Medical Device>>

The medical device includes a sliding portion. The sliding portion has a coating. The coating includes a product resulting from curing of a composition including: a hydroxy group-containing silicone resin; and at least one silane coupling agent. The composition contains an aminosilane as the at least one silane coupling agent or contains a non-aminosilane amine compound. The composition further contains a haloalkylsilane. The haloalkylsilane is a compound represented by Formula (1) below.

R¹Si(OR²)₃ (1)

In Formula (1), R¹ is a haloalkyl group having 1 or more and 6 or less carbon atoms; and
R² is an alkyl group having 1 or more and 6 or less carbon atoms.

Examples of the medical device include an injection needle with the coating on its outer surface, a bottle needle with the coating on its outer surface, an infusion tube with the coating on its outer surface, a catheter with the coating on its outer surface, a guide wire with the coating on its outer surface, a syringe barrel with the coating on its outer or inner surface, and a syringe plunger with the coating on its gasket outer surface.

The coating including a product resulting from curing of the composition will adhere well to various materials and be resistant to delamination. The silane coupling agent can form covalent bonds with active hydrogen atom-containing functional groups, such as hydroxy groups and amino groups, which exist on surfaces of various materials. The silane coupling agent can also form bonding with the hydroxy group-containing silicone resin to form a network structure. For such reasons, the coating including a product resulting from curing of the composition has lubricity, which is derived from the silicone resin, and strongly adheres to various materials.

The sliding portion may include any material. The sliding portion may include a polymer material, metal, or glass.

Examples of the polymer material include polyolefin, (meth)acrylic resin, polyester, and polyamide. Examples of polyolefin include low-density polyethylene, high-density polyethylene, polypropylene, ethylene-propylene copolymer, ethylene-vinyl acetate copolymer, poly(1-butene), and poly(4-methyl-1-pentene). Examples of (meth)acrylic resin include polymers of one or more (meth)acrylate monomers selected from the group consisting of methyl (meth)acrylate, ethyl (meth)acrylate, n-propyl (meth)acrylate, n-butyl (meth)acrylate, phenyl (meth)acrylate, and benzyl (meth)acrylate. Examples of polyester include polyethylene terephthalate (PET), polybutylene terephthalate (PBT), polyethylene naphthalate (PEN), and polylactic acid (PLA). Examples of polyamide include nylon 6, nylon 6,6, nylon 12, nylon 6,12, and MXD nylon. The polymer material is also preferably a cyclic olefin polymer (COP) which is a polymer of a cyclic olefin such as norbornene or a cyclic olefin copolymer (COC) which is a copolymer of a cyclic olefin and an olefin such as ethylene. The polymer material is also preferably any of various elastic materials. Examples of such elastic materials include natural rubber, isoprene rubber, butyl rubber, styrene-butadiene rubber, chloroprene rubber, silicone rubber, styrene elastomers, polyurethane elastomers, polyvinyl chloride elastomers, and polyolefin elastomers.

Examples of metal include various types of stainless steel, aluminum, aluminum alloys, titanium, and titanium alloys.

The surface of the sliding portion, on which the coating is to be formed, may be subjected to steam plasma treatment or any other process for introducing hydroxy groups onto the surface, so that the coating can more strongly adhere to the sliding portion when produced by curing the composition. As the number of hydroxy groups on the surface of the sliding portion increases, the coating can form more covalent bonds with the surface of the sliding portion. This results in stronger adhesion between the coating and the sliding portion.

Hereinafter, the composition for use in forming the coating will be described.

### <Composition>

As mentioned above, the composition includes a hydroxy group-containing silicone resin and at least one silane coupling agent. The composition contains an aminosilane as the at least one silane coupling agent or contains a non-aminosilane amine compound. The composition further contains a haloalkylsilane. The composition may contain only one hydroxy group-containing silicone resin or may contain a combination of two or more hydroxy group-containing silicone resins. The composition may contain only one aminosilane or may contain a combination of two or more aminosilanes. The composition may contain only one non-aminosilane amine compound or may contain a combination of two or more non-aminosilane amine compounds. The composition may contain only one haloalkylsilane or may contain a combination of two or more haloalkylsilanes.

The coating may be formed through applying the composition to the sliding portion of the medical device. For this purpose, the composition typically contains an organic solvent.

The aminosilane or the amine compound will catalyze the condensation reaction between the silane coupling agent molecules or between the silane coupling agent and the hydroxy group-containing silicone resin. Thus, the composition containing the aminosilane or the amine compound can be successfully cured to form a coating resistant to delamination from the surface of the sliding portion.

Hereinafter, essential and optional components of the composition will be described.

### [Hydroxy Group-Containing Silicone Resin]

The composition contains a hydroxy group-containing silicone resin. The hydroxy group-containing silicone resin can provide lubricity for the coating when the coating is formed using the composition. The hydroxy group-containing silicone resin can also react with the silane coupling agent (described later) to form a coating having a network structure and adhering to the sliding portion strongly.

The silicone resin may have any type of molecular structure. The silicone resin may have a linear or branched chain molecular structure. Preferably, the silicone resin has a linear chain molecular structure. The molecular chain of the silicone resin has a polyorganosiloxane structure, which is preferably a polydimethylsiloxane structure. The silicone resin may have hydroxy groups at any positions. Preferably, the silicone resin has hydroxy groups at its molecular chain ends. The silicone resin may have hydroxy groups (silanol groups) bonded to silicon atoms in its molecular chain or may have hydroxy group-containing organic groups. The hydroxy group-containing organic group is preferably a hydroxyalkyl group, more preferably a hydroxyalkyl group having 1 or more and 4 or less carbon atoms, even more preferably a hydroxyethyl or hydroxymethyl group, furthermore preferably a hydroxymethyl group. The hydroxy group-containing silicone resin is preferably a difunctional silicone resin having a linear molecular chain with two terminal silicon atoms each bonded to a hydroxy group, more preferably a modified polydimethylsiloxane having a linear molecular chain with two terminal silicon atoms each bonded to a hydroxy group.

### [Silane Coupling Agent]

The composition contains at least one silane coupling agent. The composition contains an aminosilane as the at least one silane coupling agent or contains a non-aminosilane amine compound. The composition further contains a haloalkylsilane. Specifically, the composition contains a combination of the aminosilane and the haloalkylsilane or a combination of the non-aminosilane amine compound and the haloalkylsilane. It should be noted that the composition may contain a combination of the aminosilane, the haloalkylsilane, and the non-aminosilane amine compound. The composition may also contain an additional silane coupling agent that does not correspond to the aminosilane or the haloalkylsilane as long as it will not compromise the desired effect.

### (Aminosilane)

The aminosilane may be any type of amino group-containing silane coupling agent. The aminosilane may have a primary amino group, a secondary amino group, or a tertiary amino group. The aminosilane may also have two or more amino groups. Such two or more amino groups of the aminosilane may be the same or different.

The aminosilane is preferably a compound represented by Formula (2) below.

(R⁵-(R³-NR⁷)ₐ-R⁴)-Si(OR⁶)₃ (2)

In Formula (2), R³ is an alkylene group having 1 or more and 4 or less carbon atoms;
R⁴ is an alkylene group having 1 or more and 6 or less carbon atoms;
R⁵ is a hydrogen atom or an amino group;
the amino group for R⁵ may be a primary amino group, a secondary amino group, or a tertiary amino group;
R⁶ is an alkyl group having 1 or more and 6 or less carbon atoms;
R⁷ is a hydrogen atom or an alkyl group having 1 or more and 6 or less carbon atoms; and
a is 0 or 1, provided that
when a is 0, R⁵ is an amino group.

Examples of the alkylene group for R³ include methylene, ethane-1,2-diyl (ethylene), ethane-1,1-diyl, propane-1,3-diyl, propane-1,2-diyl, and butane-1,4-diyl.

Examples of the alkylene group for R⁴ include methylene, ethane-1,2-diyl (ethylene), ethane-1,1-diyl, propane-1,3-diyl, propane-1,2-diyl, butane-1,4-diyl, pentane-1,5-diyl, and hexane-1,6-diyl.

The amino group for R⁵ may be a primary amino group, which corresponds to -NH₂. The amino group for R⁵ may be a secondary amino group. In such a case, R⁵ is preferably a monoalkylamino group. Such a monoalkylamino group preferably has an alkyl group having 1 or more and 4 or less carbon atoms. Preferred examples of the monoalkylamino group include methylamino, ethylamino, n-propylamino, isopropylamino, n-butylamino, isobutylamino, sec-butylamino, and tert-butylamino. The amino group for R⁵ may be a tertiary amino group. In such a case, R⁵ is preferably a dialkylamino group. Such a dialkylamino group preferably has an alkyl group having 1 or more and 4 or less carbon atoms. Preferred examples of the dialkylamino group include dimethylamino, diethylamino, di-n-propylamino, diisopropylamino, di-n-butylamino, diisobutylamino, di-sec-butylamino, di-tert-butylamino, methyl(ethyl)amino, methyl(n-propyl)amino, and ethyl(n-propyl)amino.

Examples of the alkyl group for R⁶ include methyl, ethyl, n-propyl, isopropyl, n-butyl, isobutyl, sec-butyl, tert-butyl, n-pentyl, and n-hexyl.

Examples of the alkyl group for R⁷ may be the same as those for the alkyl group for R⁶.

In Formula (2), a is 0 or 1, and a is preferably 1.

Preferred examples of the compound represented by Formula (2) include:
2-aminoethyltrialkoxysilanes, such as 2-aminoethyltrimethoxysilane, 2-aminoethyltriethoxysilane, and
2-aminoethyltri-n-propoxysilane;
3-aminopropyltrialkoxysilanes, such as 3-aminopropyltrimethoxysilane, 3-aminopropyltriethoxysilane, and
3-aminopropyltri-n-propoxysilane;
4-aminobutyltrialkoxysilanes, such as 4-aminobutyltrimethoxysilane, 4-aminobutyltriethoxysilane, and
4-aminobutyltri-n-propoxysilane;
N-methyl-2-aminoethyltrialkoxysilanes, such as N-methyl-2-aminoethyltrimethoxysilane, N-methyl-2-aminoethyltriethoxysilane, and N-methyl-2-aminoethyltri-n-propoxysilane;
N-methyl-3-aminopropyltrialkoxysilanes, such as N-methyl-3-aminopropyltrimethoxysilane, N-methyl-3-aminopropyltriethoxysilane, and N-methyl-3-aminopropyltri-n-propoxysilane;
N-methyl-4-aminobutyltrialkoxysilanes, such as N-methyl-4-aminobutyltrimethoxysilane, N-methyl-4-aminobutyltriethoxysilane, and N-methyl-4-aminobutyltri-n-propoxysilane;
N-ethyl-2-aminoethyltrialkoxysilanes, such as N-ethyl-2-aminoethyltrimethoxysilane, N-ethyl-2-aminoethyltriethoxysilane, and N-ethyl-2-aminoethyltri-n-propoxysilane;
N-ethyl-3-aminopropyltrialkoxysilanes, such as N-ethyl-3-aminopropyltrimethoxysilane, N-ethyl-3-aminopropyltriethoxysilane, and N-ethyl-3-aminopropyltri-n-propoxysilane;
N-ethyl-4-aminobutyltrialkoxysilanes, such as N-ethyl-4-aminobutyltrimethoxysilane, N-ethyl-4-aminobutyltriethoxysilane, and N-ethyl-4-aminobutyltri-n-propoxysilane;
N,N-dimethyl-2-aminoethyltrialkoxysilanes, such as N,N-dimethyl-2-aminoethyltrimethoxysilane, N,N-dimethyl-2-aminoethyltriethoxysilane, and N,N-dimethyl-2-aminoethyltri-n-propoxysilane;
N,N-dimethyl-3-aminopropyltrialkoxysilanes, such as N,N-dimethyl-3-aminopropyltrimethoxysilane, N,N-dimethyl-3-aminopropyltriethoxysilane, and N,N-dimethyl-3-aminopropyltri-n-propoxysilane;
N,N-dimethyl-4-aminobutyltrialkoxysilanes, such as N,N-dimethyl-4-aminobutyltrimethoxysilane, N,N-dimethyl-4-aminobutyltriethoxysilane, and N,N-dimethyl-4-aminobutyltri-n-propoxysilane;
N,N-diethyl-2-aminoethyltrialkoxysilanes, such as N,N-diethyl-2-aminoethyltrimethoxysilane, N,N-diethyl-2-aminoethyltriethoxysilane, and N,N-diethyl-2-aminoethyltri-n-propoxysilane;
N,N-diethyl-3-aminopropyltrialkoxysilanes, such as N,N-diethyl-3-aminopropyltrimethoxysilane, N,N-diethyl-3-aminopropyltriethoxysilane, and N,N-diethyl-3-aminopropyltri-n-propoxysilane;
N,N-diethyl-4-aminobutyltrialkoxysilanes, such as N,N-diethyl-4-aminobutyltrimethoxysilane, N,N-diethyl-4-aminobutyltriethoxysilane, and N,N-diethyl-4-aminobutyltri-n-propoxysilane;
N-(2-aminoethyl)-2-aminoethyltrialkoxysilanes, such as N-(2-aminoethyl)-2-aminoethyltrimethoxysilane, N-(2-aminoethyl)-2-aminoethyltriethoxysilane, and N-(2-aminoethyl)-2-aminoethyltri-n-propoxysilane;
N-(3-aminopropyl)-2-aminoethyltrialkoxysilanes, such as N-(3-aminopropyl)-2-aminoethyltrimethoxysilane, N-(3-aminopropyl)-2-aminoethyltriethoxysilane, and N-(3-aminopropyl)-2-aminoethyltri-n-propoxysilane;
N-(4-aminobutyl)-2-aminoethyltrialkoxysilanes, such as N-(4-aminobutyl)-2-aminoethyltrimethoxysilane, N-(4-aminobutyl)-2-aminoethyltriethoxysilane, and N-(4-aminobutyl)-2-aminoethyltri-n-propoxysilane;
N-(2-aminoethyl)-3-aminopropyltrialkoxysilanes, such as N-(2-aminoethyl)-3-aminopropyltrimethoxysilane, N-(2-aminoethyl)-3-aminopropyltriethoxysilane, and N-(2-aminoethyl)-3-aminopropyltri-n-propoxysilane;
N-(3-aminopropyl)-3-aminopropyltrialkoxysilanes, such as N-(3-aminopropyl)-3-aminopropyltrimethoxysilane, N-(3-aminopropyl)-3-aminopropyltriethoxysilane, and N-(3-aminopropyl)-3-aminopropyltri-n-propoxysilane;
N-(4-aminobutyl)-3-aminopropyltrialkoxysilanes, such as N-(4-aminobutyl)-3-aminopropyltrimethoxysilane, N-(4-aminobutyl)-3-aminopropyltriethoxysilane, and N-(4-aminobutyl)-3-aminopropyltri-n-propoxysilane;
N-(2-aminoethyl)-4-aminobutyltrialkoxysilanes, such as N-(2-aminoethyl)-4-aminobutyltrimethoxysilane, N-(2-aminoethyl)-4-aminobutyltriethoxysilane, and N-(2-aminoethyl)-4-aminobutyltri-n-propoxysilane;
N-(3-aminopropyl)-4-aminobutyltrialkoxysilanes, such as N-(3-aminopropyl)-4-aminobutyltrimethoxysilane, N-(3-aminopropyl)-4-aminobutyltriethoxysilane, and N-(3-aminopropyl)-4-aminobutyltri-n-propoxysilane; and
N-(4-aminobutyl)-4-aminobutyltrialkoxysilanes, such as N-(4-aminobutyl)-4-aminobutyltrimethoxysilane, N-(4-aminobutyl)-4-aminobutyltriethoxysilane, and N-(4-aminobutyl)-4-aminobutyltri-n-propoxysilane.

### (Haloalkylsilane)

The haloalkylsilane is a compound represented by Formula (1) below.

R¹Si(OR²)₃ (1)

In Formula (1), R¹ is a haloalkyl group having 1 or more and 6 or less carbon atoms; and
R² is an alkyl group having 1 or more and 6 or less carbon atoms.

The halogen atom in the haloalkyl group for R¹ may be fluorine, chlorine, bromine, or iodine and is preferably fluorine, chlorine, or bromine. The haloalkyl group for R¹ may have one type of halogen atom(s) or two or more types of halogen atoms.

Examples of the haloalkyl group for R¹ include:
monohalomethyl groups, such as chloromethyl, bromomethyl, and fluoromethyl;
dihalomethyl groups, such as dichloromethyl, dibromomethyl, and difluoromethyl;
trihalomethyl groups, such as trichloromethyl, tribromomethyl, and trifluoromethyl;
2-haloethyl groups, such as 2-chloroethyl, 2-bromoethyl, and 2-fluoroethyl;
1-haloethyl groups, such as 1-chloroethyl, 1-bromoethyl, and 1-fluoroethyl;
2,2-dihaloethyl groups, such as 2,2-dichloroethyl, 2,2-dibromoethyl, and 2,2-difluoroethyl;
1,2-dihaloethyl groups, such as 1,2-dichloroethyl, 1,2-dibromoethyl, and 1,2-difluoroethyl;
2,2,2-trihaloethyl groups, such as 2,2,2-trichloroethyl, 2,2,2-tribromoethyl, and 2,2,2-trifluoroethyl;
3-halopropyl groups, such as 3-chloropropyl, 3-bromopropyl, and 3-fluoropropyl;
2-halopropyl groups, such as 2-chloropropyl, 2-bromopropyl, and 2-fluoropropyl;
2,3-dihalopropyl groups, such as 2,3-dichloropropyl, 2,3-dibromopropyl, and 2,3-difluoropropyl;
3,3,3-trihalopropyl groups, such as 3,3,3-trichloropropyl, 3,3,3-tribromopropyl, and 3,3,3-trifluoropropyl;
2,2,3,3-tetrahalopropyl groups, such as 2,2,3,3-tetrachloropropyl, 2,2,3,3-tetrabromopropyl, and 2,2,3,3-tetrafluoropropyl;
2,2,3,3,3-pentahalopropyl groups, such as 2,2,3,3,3-pentachloropropyl, 2,2,3,3,3-pentabromopropyl, and 2,2,3,3,3-pentafluoropropyl;
1-halo-1-methylethyl groups, such as 1-chloro-1-methylethyl, 1-bromo-1-methylethyl, and 1-fluoro-1-methylethyl;
2-halo-1-methylethyl groups, such as 2-chloro-1-methylethyl, 2-bromo-1-methylethyl, and 2-fluoro-1-methylethyl;
4-halobutyl groups, such as 4-chlorobutyl, 4-bromobutyl, and 4-fluorobutyl;
5-halopentyl groups, such as 5-chloropentyl, 5-bromopentyl, and 5-fluoropentyl; and
6-halohexyl groups, such as 6-chlorohexyl, 6-bromohexyl, and 6-fluorohexyl.

The haloalkyl group for R¹ is preferably a chloroalkyl group having 1 or more and 3 or less carbon atoms. The haloalkylsilane with such a chloroalkyl group is easily available and has a relatively small molecular weight, which allows easy formation of a coating with a high cross-link density or allows easy formation of more covalent bonds between the coating and the sliding portion. The chloroalkyl group having 1 or more and 3 or less carbon atoms may have two or more chlorine atoms.

Examples of the alkyl group for R⁶ include methyl, ethyl, n-propyl, isopropyl, n-butyl, isobutyl, sec-butyl, tert-butyl, n-pentyl, and n-hexyl.

Examples of the haloalkylsilane represented by Formula (1) include:
monohalomethyltrialkoxysilanes, such as chloromethyltrimethoxysilane, bromomethyltrimethoxysilane, fluoromethyltrimethoxysilane, chloromethyltriethoxysilane, bromomethyltriethoxysilane, fluoromethyltriethoxysilane, chloromethyltri-n-propoxysilane, bromomethyltri-n-propoxysilane, and fluoromethyltri-n-propoxysilane;
dihalomethyltrialkoxysilanes, such as dichloromethyltrimethoxysilane, dibromomethyltrimethoxysilane, difluoromethyltrimethoxysilane, dichloromethyltriethoxysilane, dibromomethyltriethoxysilane, difluoromethyltriethoxysilane, dichloromethyltri-n-propoxysilane, dibromomethyltri-n-propoxysilane, and difluoromethyltri-n-propoxysilane;
trihalomethyltrialkoxysilanes, such as trichloromethyltrimethoxysilane, tribromomethyltrimethoxysilane, trifluoromethyltrimethoxysilane, trichloromethyltriethoxysilane, tribromomethyltriethoxysilane, trifluoromethyltriethoxysilane, trichloromethyltri-n-propoxysilane, tribromomethyltri-n-propoxysilane, and trifluoromethyltri-n-propoxysilane;
2-haloethyltrialkoxysilanes, such as 2-chloroethyltrimethoxysilane, 2-bromoethyltrimethoxysilane, 2-fluoroethyltrimethoxysilane, 2-chloroethyltriethoxysilane, 2-bromoethyltriethoxysilane, 2-fluoroethyltriethoxysilane, 2-chloroethyltri-n-propoxysilane, 2-bromoethyltri-n-propoxysilane, and 2-fluoroethyltri-n-propoxysilane;
1-haloethyltrialkoxysilanes, such as 1-chloroethyltrimethoxysilane, 1-bromoethyltrimethoxysilane, 1-fluoroethyltrimethoxysilane, 1-chloroethyltriethoxysilane, 1-bromoethyltriethoxysilane, 1-fluoroethyltriethoxysilane, 1-chloroethyltri-n-propoxysilane, 1-bromoethyltri-n-propoxysilane, and 1-fluoroethyltri-n-propoxysilane;
2,2-dihaloethyltrialkoxysilanes, such as 2,2-dichloroethyltrimethoxysilane, 2,2-dibromoethyltrimethoxysilane, 2,2-difluoroethyltrimethoxysilane, 2,2-dichloroethyltriethoxysilane, 2,2-dibromoethyltriethoxysilane, 2,2-difluoroethyltriethoxysilane, 2,2-dichloroethyltri-n-propoxysilane, 2,2-dibromoethyltri-n-propoxysilane, and 2,2-difluoroethyltri-n-propoxysilane;
1,2-dihaloethyltrialkoxysilanes, such as 1,2-dichloroethyltrimethoxysilane, 1,2-dibromoethyltrimethoxysilane, 1,2-difluoroethyltrimethoxysilane, 1,2-dichloroethyltriethoxysilane, 1,2-dibromoethyltriethoxysilane, 1,2-difluoroethyltriethoxysilane, 1,2-dichloroethyltri-n-propoxysilane, 1,2-dibromoethyltri-n-propoxysilane, and 1,2-difluoroethyltri-n-propoxysilane;
2,2,2-trihaloethyltrialkoxysilanes, such as 2,2,2-trichloroethyltrimethoxysilane, 2,2,2-tribromoethyltrimethoxysilane, 2,2,2-trifluoroethyltrimethoxysilane, 2,2,2-trichloroethyltriethoxysilane, 2,2,2-tribromoethyltriethoxysilane, 2,2,2-trifluoroethyltriethoxysilane, 2,2,2-trichloroethyltri-n-propoxysilane, 2,2,2-tribromoethyltri-n-propoxysilane, and 2,2,2-trifluoroethyltri-n-propoxysilane;
3-halopropyltrialkoxysilanes, such as 3-chloropropyltrimethoxysilane, 3-bromopropyltrimethoxysilane, 3-fluoropropyltrimethoxysilane, 3-chloropropyltriethoxysilane, 3-bromopropyltriethoxysilane, 3-fluoropropyltriethoxysilane, 3-chloropropyltri-n-propoxysilane, 3-bromopropyltri-n-propoxysilane, and 3-fluoropropyltri-n-propoxysilane;
2-halopropyltrialkoxysilanes, such as 2-chloropropyltrimethoxysilane, 2-bromopropyltrimethoxysilane, 2-fluoropropyltrimethoxysilane, 2-chloropropyltriethoxysilane, 2-bromopropyltriethoxysilane, 2-fluoropropyltriethoxysilane, 2-chloropropyltri-n-propoxysilane, 2-bromopropyltri-n-propoxysilane, and 2-fluoropropyltri-n-propoxysilane;
2,3-dihalopropyltrialkoxysilanes, such as 2,3-dichloropropyltrimethoxysilane, 2,3-dibromopropyltrimethoxysilane, 2,3-difluoropropyltrimethoxysilane, 2,3-dichloropropyltriethoxysilane, 2,3-dibromopropyltriethoxysilane, 2,3-difluoropropyltriethoxysilane, 2,3-dichloropropyltri-n-propoxysilane, 2,3-dibromopropyltri-n-propoxysilane, and 2,3-difluoropropyltri-n-propoxysilane;
3,3,3-trihalopropyltrialkoxysilanes, such as 3,3,3-trichloropropyltrimethoxysilane, 3,3,3-tribromopropyltrimethoxysilane, 3,3,3-trifluoropropyltrimethoxysilane, 3,3,3-trichloropropyltriethoxysilane, 3,3,3-tribromopropyltriethoxysilane, 3,3,3-trifluoropropyltriethoxysilane, 3,3,3-trichloropropyltri-n-propoxysilane, 3,3,3-tribromopropyltri-n-propoxysilane, and 3,3,3-trifluoropropyltri-n-propoxysilane;
2,2,3,3-tetrachloropropyltrimethoxysilane, 2,2,3,3-tetrabromopropyltrimethoxysilane, 2,2,3,3-tetrafluoropropyltrimethoxysilane, 2,2,3,3-tetrachloropropyltriethoxysilane, 2,2,3,3-tetrabromopropyltriethoxysilane, 2,2,3,3-tetrafluoropropyltriethoxysilane, 2,2,3,3-tetrachloropropyltriethoxysilane, 2,2,3,3-tetrabromopropyltriethoxysilane, 2,2,3,3-tetrafluoropropyltriethoxysilane, 2,2,3,3,3-pentahalopropyltrialkoxysilanes, such as 2,2,3,3,3-pentachloropropyltri-n-propoxysilane, 2,2,3,3,3-pentabromopropyltri-n-propoxysilane, and 2,2,3,3,3-pentafluoropropyltri-n-propoxysilane;
1-halo-1-methylethyltrialkoxysilanes, such as 1-chloro-1-methylethyltrimethoxysilane, 1-bromo-1-methylethyltrimethoxysilane, 1-fluoro-1-methylethyltrimethoxysilane, 1-chloro-1-methylethyltriethoxysilane, 1-bromo-1-methylethyltriethoxysilane, 1-fluoro-1-methylethyltriethoxysilane, 1-chloro-1-methylethyltri-n-propoxysilane, 1-bromo-1-methylethyltri-n-propoxysilane, and 1-fluoro-1-methylethyltri-n-propoxysilane;
2-halo-1-methylethyltrialkoxysilanes, such as 2-chloro-1-methylethyltrimethoxysilane, 2-bromo-1-methylethyltrimethoxysilane, 2-fluoro-1-methylethyltrimethoxysilane, 2-chloro-1-methylethyltriethoxysilane, 2-bromo-1-methylethyltriethoxysilane, 2-fluoro-1-methylethyltriethoxysilane, 2-chloro-1-methylethyltri-n-propoxysilane, 2-bromo-1-methylethyltri-n-propoxysilane, and 2-fluoro-1-methylethyltri-n-propoxysilane;
4-halobutyltrialkoxysilanes, such as 4-chlorobutyltrimethoxysilane, 4-bromobutyltrimethoxysilane, 4-fluorobutyltrimethoxysilane, 4-chlorobutyltriethoxysilane, 4-bromobutyltriethoxysilane, 4-fluorobutyltriethoxysilane, 4-chlorobutyltri-n-propoxysilane, 4-bromobutyltri-n-propoxysilane, and 4-fluorobutyltri-n-propoxysilane;
5-halopentyltrialkoxysilanes, such as 5-chloropentyltrimethoxysilane, 5-bromopentyltrimethoxysilane, 5-fluoropentyltrimethoxysilane, 5-chloropentyltriethoxysilane, 5-bromopentyltriethoxysilane, 5-fluoropentyltriethoxysilane, 5-chloropentyltri-n-propoxysilane, 5-bromopentyltri-n-propoxysilane, and 5-fluoropentyltri-n-propoxysilane; and 6-halohexyltrialkoxysilanes, such as 6-chlorhexyltrimethoxysilane, 6-bromhexyltrimethoxysilane, 6-fluorohexyltrimethoxysilane, 6-chlorohexyltriethoxysilane, 6-bromohexyltriethoxysilane, 6-fluorohexyltriethoxysilane, 6-chlorohexyltri-n-propoxysilane, 6-bromohexyltri-n-propoxysilane, and 6-fluorohexyltri-n-propoxysilane.

### (Additional Silane Coupling Agent)

The composition may contain any additional silane coupling agent other than the aminosilane and the haloalkylsilane as long as it will not compromise the desired effect. The content of the additional silane coupling agent in all the silane coupling agents is preferably 20% by mass or less, more preferably 10% by mass or less, even more preferably 5% by mass or less, furthermore preferably 1% by mass or less, most preferably 0% by mass.

Examples of the additional silane coupling agent include n-propyltrimethoxysilane, n-propyltriethoxysilane, n-octyltrimethoxysilane, n-octyltriethoxysilane, n-dodecyltrimethoxysilane, n-dodecyltriethoxysilane, n-hexadecyltrimethoxysilane, n-hexadecyltriethoxysilane, n-octadecyltrimethoxysilane, n-octadecyltriethoxysilane, phenyltrimethoxysilane, phenyltriethoxysilane, vinyltrimethoxysilane, vinyltriethoxysilane, allyltrimethoxysilane, allyltriethoxysilane, 1-hexenyltrimethoxysilane, 1-hexenyltriethoxysilane, 1-octenyltrimethoxysilane, 1-octenyltriethoxysilane, 3-mercaptopropyltrimethoxysilane, 3-mercaptopropyltriethoxysilane, 3-acryloyloxypropyltrimethoxysilane, 3-acryloylpropyltriethoxysilane, 3-methacryloyloxypropyltrimethoxysilane, 3-methacryloyloxypropyltriethoxysilane, 3-isocyanatopropyltrimethoxysilane, 3-isocyanatopropyltriethoxysilane, 3-glycidoxypropyltrimethoxysilane, and 3-glycidoxypropyltriethoxysilane.

The coating produced using the composition preferably includes a product of condensation reaction between 100 parts by mass of the hydroxy group-containing silicone resin and 11 parts by mass or more and 150 parts by mass or less of the silane coupling agent(s). The amount of the silane coupling agent(s) in the composition is preferably 18 parts by mass or more and 100 parts by mass or less, more preferably 25 parts by mass or more and 43 parts by mass or less, based on 100 parts by mass of the hydroxy group-containing silicone resin. The coating formed by condensation reaction between the hydroxy group-containing silicone resin and the silane coupling agent(s) in such a mass ratio tends to have a high cross-link density and tends to strongly adhere to the sliding portion.

In a case where the composition contains the aminosilane and the haloalkylsilane as the silane coupling agents, the ratio of the mass of the haloalkylsilane to the total mass of the haloalkylsilane and the aminosilane is preferably 20% by mass or more and 85% by mass or less, more preferably 25% by mass or more and 80% by mass or less, even more preferably 30% by mass or more and 75% by mass or less.

### [Amine Compound]

The amine compound may be any type commonly used as a catalyst for the condensation reaction of conventional silane coupling agents or silane compounds having a hydrolysable group such as an alkoxy group.

The amine compound may be an aliphatic primary amine, an aliphatic secondary amine, an aliphatic tertiary amine, an aromatic amine, a nitrogen-containing heterocyclic compound, an amidine structure-containing compound, a guanidine structure-containing compound, a biguanide structure-containing compound, or any amine other than the foregoing.

Examples of the amine compound include:
aliphatic primary amines, such as methylamine, ethylamine, n-propylamine, isopropylamine, n-butylamine, n-pentylamine, n-hexylamine, n-octylamine, 2-ethylhexylamine, n-nonylamine, n-decylamine, laurylamine, n-pentadecylamine, cetylamine, stearylamine, cyclohexylamine, oleylamine, monoethanolamine, 3-hydroxypropylamine, ethylenediamine, propylenediamine, hexamethylenediamine, and 3-methoxypropylamine;
aliphatic secondary amines, such as dimethylamine, diethylamine, di-n-propylamine, diisopropylamine, di-n-butylamine, di-n-pentylamine, di-n-hexylamine, di-n-octylamine, di(2-ethylhexyl)amine, di-n-decylamine, dilaurylamine, dicetylamine, distearylamine, methylstearylamine, ethylstearylamine, butylstearylamine, diethanolamine, and N,N'-dimethyl-1,3-propanediamine;
aliphatic tertiary amines, such as triethylamine, diisopropylethylamine, tri-n-butylamine, tri-n-hexylamine, tri-n-octylamine, triallyamine, and triethanolamine;
aromatic amines, such as aniline, laurylaniline, stearylaniline, and triphenylamine;
nitrogen-containing heterocyclic compounds, such as pyridine, 2-aminopyridine, 2-(dimethylamino)pyridine, 4-(dimethylamino)pyridine, 2-hydroxypyridine, imidazole, 2-ethyl-4-methylimidazole, morpholine, N-methylmorpholine, piperidine, 2-piperidinemethanol, 2-(2-piperidino)ethanol, piperidone, 1,2-dimethyl-1,4,5,6-tetrahydropyrimidine, 1,4-diazabicyclo[2.2.2]octane (DABCO), and aziridine;
amidine structure-containing compounds, such as 1,8-diazabicyclo[5.4.0]undeca-7-ene (DBU), 6-(dibutylamino)-1,8-diazabicyclo[5.4.0]undeca-7-ene (DBA-DBU), 6-(2-hydroxypropyl)-1,8-diazabicyclo[5.4.0]undeca-7-ene (OH-DBU), and 1,5-diazabicyclo[4.3.0]nona-5-ene (DBN);
guanidine structure-containing compounds, such as guanidine, phenylguanidine, and diphenylguanidine; and
biguanide structure-containing compounds, such as butylbiguanide, 1-o-tolylbiguanide, and 1-phenylbiguanide.

The composition may contain any amount of the amine compound as long as it will not compromise the desired effect. The composition preferably contains 6 parts by mass or more and 30 parts by mass or less, more preferably 10 parts by mass or more and 20 parts by mass or less of the amine compound, based on 100 parts by mass of the silane coupling agent(s).

### [Organic Solvent]

The composition preferably contains an organic solvent so that it can be applied to medical devices. The organic solvent may be any type capable of well dissolving the components described above.

For dissolution of the hydroxy group-containing silicone resin, the organic solvent may be hydrochlorofluoroolefin (HCFO). Preferred examples of hydrochlorofluoroolefin include AMOLEA^{®} AS-300 ((E)-1-chloro-2,3,3-trifluoropropene/(Z)-1-chloro-2,3,3-trifluoropropene manufactured by AGC) and CELEFIN^{®} 1233Z (manufactured by Honcho Chemical). Chlorinated hydrocarbon solvents other than hydrochlorofluoroolefin, such as dichloromethane, chloroform, 1,2-dichloroethane, 1,1-dichloroethane, and carbon tetrachloride, are also preferable in terms of the solubility of the hydroxy group-containing silicone resin.

For dissolution of the silane coupling agent, the organic solvent is preferably an alkanol. Examples of the alkanol include methanol, ethanol, n-propanol, isopropanol, n-butanol, n-pentanol, and n-hexanol.

The composition is preferably prepared by mixing a hydrochlorofluoroolefin solution of the hydroxy group-containing silicone resin or a chlorinated hydrocarbon (not hydrochlorofluoroolefin) solution of the hydroxy group-containing silicone resin with an alkanol solution of the silane coupling agent. The amine compound may be added to the hydroxy group-containing silicone resin solution or the silane coupling agent solution. Alternatively, the amine compound may be added to a mixture of the hydroxy group-containing silicone resin solution and the silane coupling agent solution.

The composition may contain any suitable amount of the organic solvent. The content of the organic solvent in the composition may be selected as appropriate depending on the thickness of the coating to be formed using the composition and any other factor. The composition with higher contents of the hydroxy group-containing silicone resin and the silane coupling agent can easily form a thicker coating.

### [Water]

The composition can be cured upon exposure to moisture in the air. Therefore, the composition may contain no water. However, the composition preferably contains water so that it can undergo rapid curing. The amount of water in the composition is preferably 10 parts by mass or more and 50 parts by mass or less, more preferably 20 parts by mass or more and 35 parts by mass or less, based on 100 parts by mass of the silane coupling agent(s).

### <Method for Forming the Coating>

The composition described above is applied to the surface of a sliding portion of a medical device to form the coating. The composition may be applied using any suitable method. The composition may be applied using a dipping method, a spray method, an inkjet printing method, a screen printing method, a roll coating method, or any other coating method. In particular, the composition is preferably applied using a dipping method, which allows easy application of the composition to the surface of a medical device with a complicated shape. Alternatively, the composition is preferably applied using a spray method, which can prevent volatilization of the organic solvent and prevent changes in the concentration of the composition. The composition applied to the medical device may be cured using any suitable method. The composition may be cured at room temperature. Alternatively, the composition may be cured under heating conditions for acceleration of the curing. The composition may be heated at any suitable temperature that will not cause thermal degradation or deformation of the material of the medical device or will not cause decomposition or thermal degradation of the components in the coating. For example, the composition is preferably heated at a temperature of 50°C or more and 200°C or less, more preferably 60°C or more and 190°C or less, even more preferably 80°C or more and 180°C or less. The heating time may be selected as appropriate depending on the heating temperature and any other factor. Typically, the composition is preferably heated for a time period of 5 minutes or more and 6 hours or less, more preferably 10 minutes or more and 5 hours or less, even more preferably 30 minutes or more and 2 hours or less.

The production process described above produces the medical device having the sliding portion that exhibits excellent glide properties. In the resulting medical device, the coating on the sliding portion is resistant to delamination, which allows the medical device to maintain high glide properties for a long time and minimizes the adverse effect of coating delamination on drug solutions or living bodies.

### <<Syringe>>

The syringe includes a barrel and a plunger. The barrel typically has a luer at one end and an opening at the other end. Through the luer, a fluid is allowed to flow into or out of the barrel. The luer has an opening through which the fluid passes. The plunger includes a plunger rod and a gasket. The plunger is inserted into the barrel through the opening opposite the luer of the barrel. When in contact with the inner surface of the barrel, the gasket seals the internal space of the barrel. The gasket can seal the internal space of the barrel when it is moved, inside the barrel, together with the plunger rod being pushed or pulled.

The barrel and the gasket of the plunger are preferably made of a polymer material, which is lightweight and resistant to breaking and on which a highly adhesive coating can be easily formed. Examples of the polymer material may be the same as those listed above.

The syringe has the above-mentioned coating on at least one selected from the group consisting of the inner surface of the barrel and the outer surface of the gasket. The coating allows the plunger to move smoothly with a small force in the syringe.

The syringe is usually used to contain various drug solutions or liquid biological samples inside the barrel. In the syringe, the coating, which is resistant to delamination from the inner surface of the barrel or the outer surface of the gasket, has less adverse effect on the drug solution or the liquid biological sample in the barrel.

### <<Prefilled Syringe>>

The prefilled syringe includes a syringe barrel, a syringe plunger, a sealant, and a drug. The drug is contained in the barrel. The plunger has a gasket. One opening of the barrel is sealed with the gasket. The other opening of the barrel is sealed with the sealant.

The barrel has a luer at one end and an opening at the other end. Through the luer, a fluid is allowed to flow into or out of the barrel. The luer has an opening through which the fluid passes. In the prefilled syringe, the opening of the luer is sealed with the sealant.

The plunger has the structure described above.

The drug in the barrel is in the form of a liquid. Examples of the drug include a physiological saline solution, a heparin saline solution, a contrast agent, a hyaluronic acid formulation, an insulin formulation, a rheumatoid medication, and an anticancer agent.

The prefilled syringe has the above-mentioned coating on at least one selected from the group consisting of the inner surface of the barrel and the outer surface of the gasket. The coating allows the plunger to move smoothly with a small force in the prefilled syringe.

In the prefilled syringe, the coating is also resistant to delamination from the inner surface of the barrel or the outer surface of the gasket. Thus, the coating has less adverse effect on the drug in the barrel.

### EXAMPLES

### [Example 1]

A hydroxy group-containing silicone resin solution was prepared by dissolving 40 parts by mass of modified dimethylpolysiloxane having hydroxy groups at both ends of its linear molecular chain in 783 parts by mass of AMOLEA^{®} AS-300 (manufactured by AGC). A silane coupling agent solution was prepared by dissolving 30 parts by mass of chloromethyltrimethoxysilane and 30 parts by mass of N-(2-aminoethyl)-2-aminoethyltrimethoxysilane in 783 parts by mass of isopropanol. The silane coupling agent solution was added dropwise to the hydroxy group-containing silicone resin solution being stirred with a magnetic stirrer. After the dropwise addition of the silane coupling agent solution was completed, 17 parts by mass of water was added to the mixture of the hydroxy group-containing silicone resin solution and the silane coupling agent solution to form a coating composition. A gasket for a 2.25 mL syringe plunger was dipped for 5 seconds in the resulting composition for the formation of a coating film on the surface of the gasket. The gasket coated with the composition was air-dried at room temperature and then heated at 145°C for 120 minutes so that the composition formed a coating.

### [Examples 2 to 16 and Comparative Examples 1 to 7]

Compositions were prepared as in Example 1 except that the amount of the hydroxy group-containing silicone resin used was as shown in Table 1 and that the type and amount of the silane coupling agent used were as shown in Table 1. The composition obtained in each of the examples and the comparative examples was applied to the surface of the gasket for a 2.25 mL syringe plunger and then cured to form a coating as in Example 1.

### [Example 17]

A composition was prepared as in Example 1 except that the amount of the hydroxy group-containing silicone resin used was as shown in Table 1, the type and the amount of the silane coupling agent used were as shown in Table 1, and the composition further contained 11 parts by mass of triethylamine as the amine compound. The resulting composition was applied to the surface of a gasket for a 2.25 mL syringe plunger and then cured to form a coating as in Example 1.

The types of the silane coupling agents listed in Table 1 are as follows.

### (Haloalkylsilane Coupling Agents)

A: Chloromethyltrimethoxysilane
B: 3-Chloropropyltrimethoxysilane
C: 3-Trifluoropropyltrimethoxysilane
D: 3-Bromopropyltrimethoxysilane

### (Aminosilane Coupling Agents)

a: N-(2-aminoethyl)-2-aminoethyltrimethoxysilane
b: 3-Aminopropyltrimethoxysilane
c: N-methyl-3-aminopropyltrimethoxysilane
d: N,N-dimethyl-3-aminopropyltrimethoxysilane

### (Additional Silane Coupling Agents)

I: 3-Glycidoxypropyltrimethoxysilane
II: Methyltrimethoxysilane

The gasket with the coating on its surface, prepared in each of the examples and the comparative example, was used for the preparation of a syringe. The syringe was subjected to the method described below fossr evaluating the glide properties of the gasket in the barrel. It was also evaluated how much the coating could delaminate from the gasket prepared in each of the examples and the comparative examples. The evaluation results are shown in Table 2.

### <Evaluation of Glide Properties of Gaskets>

The gasket was inserted into a COP barrel with a volume of 2.25 mL. The gasket was placed at the nominal syringe volume position. Water was injected and charged into the barrel through its luer. A rubber cap was attached to the luer of the barrel, and a plunger rod was attached to the gasket, so that a syringe was prepared. The resulting syringe filled with water was allowed to stand at 20 to 30°C for 24 hours. An autograph (AG-X plus manufactured by Shimadzu) equipped with a load cell (SLBL-500N manufactured by Shimadzu) was used to measure the load applied when the plunger was pushed until the gasket was shifted at a rate of 300 mm/minute to a position 5 mm from the top of the barrel, from which a load-stroke chart was obtained. The rubber cap was detached from the syringe before the measurement using the load cell. The initial glide force (N) and the maximum glide force (N) were read from the resulting load-stroke chart. The load-stroke chart shows a peak at which the load reaches a maximum during the start stage of pushing the plunger. The maximum load value at this peak corresponds to the initial glide force (N). The maximum glide force (N) is determined as the maximum load applied when the plunger displacement exceeds the displacement range corresponding to the peak. If the plunger of the syringe is pushed manually, the feeling of how easily the plunger can be pushed will be more influenced by the initial glide force than by the maximum glide force. This is because once the plunger begins to move, it continues to move even if the maximum glide force is relatively large. The glide properties of the gasket were evaluated according to the criteria below based on the initial and maximum glide forces. The evaluation results are shown in Table 2.

### (Initial Glide Force)

⊙ (Excellent): The initial glide force is 6 N or less.
∘ (Good): The initial glide force is 8 N or less.
× (Poor): The initial glide force is more than 8 N.

### (Maximum Glide Force)

⊙ (Excellent): The maximum glide force is 10 N or less.
∘ (Good): The maximum glide force is 15 N or less.
× (Poor): The maximum glide force is more than 15 N.

### <Measurement of the Amount of Coating Delamination>

Four of the gaskets with the coating on their surface were placed in a 50 mL volumetric flask, which was then closed with its lid. The gaskets in the volumetric flask were shaken under 40 rpm conditions for 3 hours using a shaker (R2200 manufactured by Argos Technologies). After the gaskets were removed from the volumetric flask, 2 mL of methyl isobutyl ketone was added to the volumetric flask, whereby the delaminated portion of the coating was solved in the methyl isobutyl solution in the volumetric flask. The methyl isobutyl ketone solution sample from the volumetric flask was subjected to ICP emission spectrometry for the quantification of silicon atoms derived from the delaminated portion of the coating from the gasket. The amount of coating delamination from the four gaskets was calculated using the measured quantity of silicon atoms and the calculated silicon atom content per unit mass of the cured composition of the coating in each of the examples and the comparative examples. The calculated amount of coating delamination from the four gaskets was divided by 4 to yield the amount of coating delamination from one gasket (average value). Table 2 shows the amount of coating delamination from one gasket (average value). The calculated amount (µg per one gasket) of coating delamination was used to evaluate the resistance of the coating to delamination according to the criteria below. The evaluation results are shown in Table 2.
⊙ (Very high): The amount of silicon atoms derived from coating delamination from the gasket is 20 µg or less per gasket.
∘ (High): The amount of silicon atoms derived from coating delamination from the gasket is 45 µg or less per gasket.
× (Low): The amount of silicon atoms derived from coating delamination from the gasket is more than 45 µg per gasket.

**[Table 1]**

| | Hydroxy group-containing silicone resin (parts by mass) | Haloalkylsilane coupling agent (type/parts by mass) | Aminosilane coupling agent (type/parts by mass) | Additional coupling agent (type/parts by mass) | Amine compound (parts by mass) |
|---|---|---|---|---|---|
| Example 1 | 40 | A/30 | a/30 | - | - |
| Example 2 | 50 | A/25 | a/25 | - | - |
| Example 3 | 60 | A/20 | a/20 | - | - |
| Example 4 | 70 | A/10 | a/20 | - | - |
| Example 5 | 70 | A/15 | a/15 | - | - |
| Example 6 | 70 | A/20 | a/10 | - | - |
| Example 7 | 80 | A/5 | a/15 | - | - |
| Example 8 | 80 | A/10 | a/10 | - | - |
| Example 9 | 80 | A/15 | a/5 | - | - |
| Example 10 | 90 | A/5 | a/5 | - | - |
| Example 11 | 80 | B/10 | a/10 | - | - |
| Example 12 | 80 | C/10 | a/10 | - | - |
| Example 13 | 80 | D/10 | a/10 | - | - |
| Example 14 | 80 | A/10 | b/10 | - | - |
| Example 15 | 80 | A/10 | c/10 | - | - |
| Example 16 | 80 | A/10 | d/10 | - | - |
| Example 17 | 80 | A/20 | - | - | 11 |
| Comparative Example 1 | 80 | - | a/20 | - | - |
| Comparative Example 2 | 80 | A/20 | - | - | - |
| Comparative Example 3 | 80 | B/20 | - | - | - |
| Comparative Example 4 | 80 | C/20 | - | - | - |
| Comparative Example 5 | 80 | D/20 | - | - | - |
| Comparative Example 6 | 80 | A/10 | - | I/10 | - |
| Comparative Example 7 | 80 | A/10 | - | II/10 | - |

**[Table 2]**

| | Initial glide force (N)/rating | Maximum glide force (N) /rating | Amount of coating delamination (µg/gasket) /rating |
|---|---|---|---|
| Example 1 | 6.10/○ | 14.48/○ | 1.7/⊙ |
| Example 2 | 5.29/⊙ | 9.95/⊙ | 1.7/⊙ |
| Example 3 | 4.39/⊙ | 7.80/⊙ | 3.7/⊙ |
| Example 4 | 5.37/⊙ | 8.20/⊙ | 17.1/⊙ |
| Example 5 | 4.42/⊙ | 7.11/⊙ | 8.0/⊙ |
| Example 6 | 4.59/⊙ | 7.52/⊙ | 5.7/⊙ |
| Example 7 | 5.65/⊙ | 8.17/⊙ | 27.0/○ |
| Example 8 | 4.39/⊙ | 7.57/⊙ | 17.3/⊙ |
| Example 9 | 4.66/⊙ | 6.00/⊙ | 10.0/⊙ |
| Example 10 | 4.58/⊙ | 7.69/⊙ | 36.2/○ |
| Example 11 | 5.17/⊙ | 7.47/⊙ | 24.1/○ |
| Example 12 | 6.61/○ | 8.97/⊙ | 3.9/⊙ |
| Example 13 | 4.63/⊙ | 7.56/⊙ | 44.1/○ |
| Example 14 | 4.96/⊙ | 7.85/⊙ | 5.3/⊙ |
| Example 15 | 4.54/⊙ | 8.16/⊙ | 5.6/⊙ |
| Example 16 | 7.25/○ | 9.36/⊙ | 5.1/⊙ |
| Example 17 | 5.80/⊙ | 7.65/⊙ | 20.0/⊙ |
| Comparative Example 1 | 6.14/○ | 7.77/⊙ | 45.9/× |
| Comparative Example 2 | 12.18/× | 7.39/⊙ | 153.9/× |
| Comparative Example 3 | 16.03/× | 13.98/○ | 120.0/× |
| Comparative Example 4 | 12.14/× | 14.07/○ | 125.0/× |
| Comparative Example 5 | 11.21/× | 13.75/○ | 138.8/× |
| Comparative Example 6 | 15.52/× | 11.61/○ | 124.4/× |
| Comparative Example 7 | 9.74/× | 11.99/○ | 71.9/× |

Tables 1 and 2 show that the gasket of each of the examples with the coating produced using the composition satisfying the specified conditions has high glide properties in the syringe and that the coating is resistant to delamination. On the other hand, the glide properties of the gasket and/or the delamination resistance of the coating was poor in the syringes having the gaskets with the coatings produced in the comparative examples using the compositions not satisfying the specified conditions.

## Claims

1. A medical device comprising a sliding portion having a coating,
the coating comprising a product resulting from curing of a composition comprising: a hydroxy group-containing silicone resin; and at least one silane coupling agent,
the composition containing an aminosilane as the at least one silane coupling agent or containing a non-aminosilane amine compound,
the at least one silane coupling agent including a haloalkylsilane represented by Formula (1):
R¹Si(OR²)₃ (1)
wherein R¹ is a haloalkyl group having 1 or more and 6 or less carbon atoms, and R² is an alkyl group having 1 or more and 6 or less carbon atoms.

2. The medical device according to claim 1,
wherein the composition contains the aminosilane, and wherein the aminosilane comprises a silane compound represented by Formula (2):
(R⁵-(R³-NR⁷)ₐ-R⁴)-Si(OR⁶) ₃ (2)
wherein R³ is an alkylene group having 1 or more and 4 or less carbon atoms, R⁴ is an alkylene group having 1 or more and 6 or less carbon atoms, R⁵ is a hydrogen atom or an amino group, the amino group for R⁵ may be a primary amino group, a secondary amino group, or a tertiary amino group, R⁶ is an alkyl group having 1 or more and 6 or less carbon atoms, R⁷ is a hydrogen atom or an alkyl group having 1 or more and 6 or less carbon atoms, and a is 0 or 1, provided that when a is 0, R⁵ is an amino group.

3. The medical device according to claim 1 or 2, wherein the medical device is an injection needle with the coating on its outer surface, a bottle needle with the coating on its outer surface, an infusion tube with the coating on its outer surface, a catheter with the coating on its outer surface, a guide wire with the coating on its outer surface, a syringe barrel with the coating on its outer or inner surface, or a syringe plunger with the coating on an outer surface of a gasket.

4. The medical device according to claim 1 or 2, wherein the coating comprises a product resulting from condensation reaction between 100 parts by mass of the silicone resin and 11 parts by mass or more and 150 parts by mass or less of the at least one silane coupling agent.

5. The medical device according to claim 1 or 2, wherein the ratio of the mass of the haloalkylsilane to the total mass of the haloalkylsilane and the aminosilane is 20% by mass or more and 85% by mass or less.

6. The medical device according to claim 1 or 2, wherein R¹ is a chloroalkyl group having 1 or more and 3 or less carbon atoms.

7. The medical device according to claim 1 or 2, wherein a is 1.

8. A syringe comprising: a barrel; and a plunger, wherein the barrel is the syringe barrel as the medical device according to claim 3 or the plunger is the syringe plunger as the medical device according to claim 3.

9. The syringe according to claim 8, wherein the syringe barrel or the gasket of the syringe plunger comprises a polymer material.

10. A prefilled syringe comprising: a syringe barrel; a syringe plunger; a sealant; and a drug,
the drug being contained in the barrel,
the syringe plunger having a gasket,
the syringe barrel having an opening sealed with the gasket,
the syringe barrel having another opening sealed with the sealant,
at least one selected from the group consisting of an inner surface of the syringe barrel and a surface of the gasket having a coating,
the coating comprising a product resulting from curing of a composition comprising: a hydroxy group-containing silicone resin; and at least one silane coupling agent,
the composition containing an aminosilane as the at least one silane coupling agent or containing a non-aminosilane amine compound,
the at least one silane coupling agent including a haloalkylsilane represented by Formula (1):
R¹Si(OR²)₃ (1)
wherein R¹ is a haloalkyl group having 1 or more and 6 or less carbon atoms, and R² is an alkyl group having 1 or more and 6 or less carbon atoms.

11. The prefilled syringe according to claim 10,
wherein the composition contains the aminosilane, and
wherein the aminosilane comprises a silane compound represented by Formula (2):
(R⁵-(R³-NR⁷)ₐ-R⁴)-Si(OR⁶)₃ (2)
wherein R³ is an alkylene group having 1 or more and 4 or less carbon atoms, R⁴ is an alkylene group having 1 or more and 6 or less carbon atoms, R⁵ is a hydrogen atom or an amino group, the amino group for R⁵ may be a primary amino group, a secondary amino group, or a tertiary amino group, R⁶ is an alkyl group having 1 or more and 6 or less carbon atoms, R⁷ is a hydrogen atom or an alkyl group having 1 or more and 6 or less carbon atoms, and a is 0 or 1, provided that when a is 0, R⁵ is an amino group.

12. A treatment liquid for use in forming a coating on a sliding portion of a medical device,
the treatment liquid comprising: a hydroxy group-containing silicone resin; and at least one silane coupling agent,
the treatment liquid containing an aminosilane as the at least one silane coupling agent or containing a non-aminosilane amine compound,
the at least one silane coupling agent including a haloalkylsilane represented by Formula (1):
R¹Si(OR²)₃ (1)
wherein R¹ is a haloalkyl group having 1 or more and 6 or less carbon atoms, and R² is an alkyl group having 1 or more and 6 or less carbon atoms.

13. The treatment liquid according to claim 12,
wherein the treatment liquid contains the aminosilane, and
wherein the aminosilane comprises a silane compound represented by Formula (2):
(R⁵-(R³-NR⁷)ₐ-R⁴)-Si(OR⁶)₃ (2)
wherein R³ is an alkylene group having 1 or more and 4 or less carbon atoms, R⁴ is an alkylene group having 1 or more and 6 or less carbon atoms, R⁵ is a hydrogen atom or an amino group, the amino group for R⁵ may be a primary amino group, a secondary amino group, or a tertiary amino group, R⁶ is an alkyl group having 1 or more and 6 or less carbon atoms, R⁷ is a hydrogen atom or an alkyl group having 1 or more and 6 or less carbon atoms, and a is 0 or 1, provided that when a is 0, R⁵ is an amino group.

14. A method for producing a medical device having a sliding portion,
the method comprising: applying the treatment liquid according to claim 12 or 13 to the sliding portion of the medical device to form a coating.
